# EUROPEAN PATENT APPLICATION

(11) **EP 2 679 283 A2**
(43) Date of publication of application: **01.01.2014**
(21) Application number: 13173988.0
(22) Date of filing: 27.06.2013
(51) Int. Cl.: A62B 7/14

(54) **Aircraft cabin with zonal OBOGS oxygen supply**

(30) Priority: 28.06.2012 US 201261665486 P
(71) Applicant: INTERTECHNIQUE, 78373 Plaisir Cedex (FR)
(72) Inventor: Rittner, Wolfgang, 23623 Ahrensbök (DE); Meckes, Rüdiger, 23919 Berkenthin (DE); Boomgarden, Günter, 23684 Scharbeutz (DE); Hollm, Marco, 25548 Rosdorf (DE)
(74) Representative: Eisenführ Speiser

(57) **Abstract**

The invention relates to an oxygen supply device for aircraft inhabitants, comprising a first On-Board Oxygen System (OBOGS) unit connected to a first set of oxygen masks, wherein a second On-Board Oxygen System (OBOGS) unit connected to a second set of oxygen masks.

## Description

The invention relates to an oxygen supply device for aircraft inhabitants, comprising a first On-Board Oxygen System (OBOGS) unit connected to a first set of oxygen masks. The invention further relates to a civil aircraft, comprising an aircraft inhabitant cabin with a plurality of seats for the aircraft inhabitants, and at least one device of the above mentioned type. The invention further relates to an OBOGS unit for use in such a device and such a civil aircraft.

In civil aircraft the safety of the inhabitants is a crucial issue. In case of a cabin decompression or cabin pressure drop inside a cabin of an aircraft the inhabitants usually are supplied with oxygen from emergency oxygen devices. Such devices usually comprise an oxygen source and at least one oxygen mask. The oxygen mask is usually stored in a ceiling part of the cabin inside a passenger service unit (PSU), which further may comprise air gaspers, reading light and further units.

As for oxygen sources different types are available on the market. Gaseous compressed oxygen storages are often used. However because of the high pressure the storage barrels have a certain weight and the amount of oxygen is limited to the volume of the storage. A second type is chemical oxygen generators (COG), which use a chemical reaction to produce gaseous oxygen out of a solid substance containing oxygen in chemically bound from.

In recent times so called On-Board Oxygen System (OBOGS) units have been used. Such OBOGS units use a microcellular sieve to filter the nitrogen out of the air to supply oxygen enriched breathable air. They are able to literally "produce its own oxygen". It is possible to produce output gases which are enriched up to 95% percent of oxygen. Such an OBOGS unit is known to supply oxygen to pilots of military aircraft.

The object of the present invention is to provide a device of the above mentioned type, in particular an oxygen supply device for aircraft inhabitants, which involves improved safety measures. One particular object of the invention is to provide such a device which is safer in case of device failure. The object of the present invention is further to provide a civil aircraft of the above mentioned type which is improved with respect to safety of the oxygen supply system.

The invention starts from the consideration that safety of an emergency oxygen system should motivate a significant improve; thus a technical concept should be found wherein it is possible to design an oxygen supply device for aircraft inhabitants which is safety improved in case of failure of an OBOGS unit. The proposed concept of the invention has arisen from the desire to increase safety with at the same time keeping the additional weight at a low level or to reduce the weight of the emergency oxygen system. The invention proposes to comprise a second On-Board Oxygen System (OBOGS) unit connected to a second set of oxygen masks.

The invention recognizes that a particular combination of two OBOGS units improves the safety of an oxygen supply device significantly. Preferably each mask is only connected to one OBOGS unit. Hence, the oxygen masks of the first set of oxygen masks are only connected to the first OBOGS unit and the oxygen masks of the second set of oxygen masks are only connected to the second OBOGS unit. In a preferred embodiment, each set of oxygen masks is provided for a specific zone inside an aircraft cabin, e.g. a specific group of seats. For example the first set may be provided for the business class and the second set may be provided for the economy class. Different arrangements can be imagined. Therefore, in case one of the OBOGS units does not produce the desired amount of oxygen the inhabitants may receive their oxygen from the second OBOGS unit. Preferably each oxygen mask is connected via a wire, tube, hose, connection line or similar devices with the associated OBOGS unit to guide the oxygen from the OBOGS unit to the oxygen mask. With this arrangement the overall wiring, tubing or lining from the OBOGS unit to the associated oxygen mask can be reduced compared to common systems. The whole oxygen supply device is preferably adapted to be arranged in a ceiling portion of a cabin of a civil aircraft. The oxygen masks are preferably adapted to be held in PSUs above the aircraft inhabitants.

These and further developed configurations of the invention are further outlined in the dependent claims. Thereby, the mentioned advantages of the proposed concept are even more improved. For each feature of the dependent claims it is claimed independent protection independent from all other features of this disclosure.

In a particular preferred development the second OBOGS unit is connected to at least one oxygen mask out of the first set of oxygen masks. Preferably also the first OBOGS unit is connected to at least one oxygen mask out of the second set of oxygen masks. Further preferred, each oxygen mask is only connected to one OBOGS unit. Due to this arrangement, the zones defined by the OBOGS unit and set of oxygen masks are not separated strictly, but are interlinked or interconnected. Therefore, in the exemplary case the first OBOGS unit suffers from a defect and is not able to supply the desired amount of oxygen, there is at least one oxygen mask in the first set of oxygen masks which is supplied with oxygen from the second OBOGS unit.

Further preferably a third On-Board Oxygen System (OBOGS) unit is connected to a third set of oxygen masks, wherein the third OBOGS unit is connected to at least one oxygen mask out of the second set of oxygen masks. Preferably the first OBOGS unit is further connected to at least one oxygen mask out of the third set of oxygen masks. This leads to an additional redundancy and may improve the overall safety of the oxygen supply device for aircraft inhabitants. Additional OBOGS units connected with additional sets of oxygen masks, e.g. a fourth, fifth etc. OBOGS units and associated sets of oxygen masks can be comprised in the oxygen supply device and interlinked with the other set of oxygen masks as set forth above.

According to such embodiments it is preferred and advantageous that the second and/or the third OBOGS unit is connected to a group of oxygen masks, preferably every second oxygen mask, out of the first and/or third set of oxygen masks respectively. Thus in one alternative the oxygen supply device comprises first and second OBOGS units connected with first and second sets of oxygen masks. A group of oxygen masks, preferably every second oxygen mask, out of the first set of oxygen masks is connected to the second OBOGS unit and preferably a similar group of oxygen masks, preferably every second oxygen mask, out of the second set of oxygen masks is connected to the first OBOGS unit. In an alternative in which the oxygen supply device comprises three OBOGS units each connected with a set of oxygen masks, it is preferred that additionally the third OBOGS unit is connected to a group of oxygen masks, preferably every second oxygen mask, out of the second set of oxygen masks und the first OBOGS unit is connected a group of oxygen masks, preferably every second oxygen mask, out of the third set of oxygen masks. It should be understood that it is possible and advantageous to comprise even more OBOGS units connected with sets of oxygen masks in the device. The interlinked connection of the different sets of oxygen masks and OBOGS units should be organized in a similar way. This leads to even better redundancies in the oxygen supply for the aircraft inhabitants. For example the oxygen masks with odd order numbers out of the first set of oxygen masks are connected to the first OBOGS unit and the oxygen masks with even order numbers out of the first set of oxygen masks are connected to the second OBOGS unit. Therefore, in case one OBOGS suffers from a defect, aircraft inhabitants may share their oxygen masks with neighbours whose oxygen masks are connected to a different OBOGS unit.

According to a further preferred embodiment, the first OBOGS unit is connected to the second OBOGS unit for supplying oxygen from the first OBOGS unit to the second OBOGS unit. Preferably also the second OBOGS unit is connected to the first OBOGS unit for supplying oxygen from the second OBOGS unit to the first OBOGS unit. In such an embodiment in one alternative the oxygen masks of the set are only connected to the associated OBOGS unit, i.e. the oxygen masks out of the first set of oxygen masks are only connected to the first OBOGS unit and the oxygen masks out of the second set of oxygen masks are only connected to the second OBOGS unit. Due to such an embodiment it becomes possible to supply oxygen from one OBOGS unit to another OBOGS unit and from the receiving OBOGS unit to the connected oxygen masks in case of failure of the receiving OBOGS unit. Preferably a valve is arranged between two connected OBOGS units, to control the amount of oxygen supplied from one OBOGS unit to another OBOGS unit. Such a valve may be connected with an activator, connected to a sensor which senses a failure or defect of one OBOGS unit. In case the sensor detects a failure, the valve may be activated by means of the activator to establish the oxygen supply from the working OBOGS unit to the defect OBOGS unit. The valve may also be activated by means ob a switch or the like, so that the cabin crew or any authorised inhabitant can establish the oxygen supply. Additionally in other embodiments, the second OBOGS unit may further be connected to at least one oxygen mask out of the first set of oxygen masks and/or the first OBOGS unit may further be connected to at least one oxygen mask out of the second set of oxygen masks. Further OBOGS unit and associated sets of oxygen masks may also be comprised in devices according to these embodiments. Such a redundancy leads to improved safety of an oxygen supply system and may also reduce the total wiring, tubing or lining compared to conventional systems.

According to another particular preferred embodiment, each OBOGS unit and/or each set of oxygen masks is dedicated to one defined zone inside a cabin of a civil aircraft and each zone includes a substantially similar number of seats for inhabitants. Thus all OBOGS units of the oxygen supply device can be designed to produce the same amount of oxygen. By defining specific zones with dedicated OBOGS units and sets of oxygen masks it becomes easier to construct the oxygen supply device with increased safety measures. Additional, it becomes easy for the cabin crew to detect a failure of one OBOGS unit and thereby finding an alternative solution, such as inhabitants sharing oxygen masks.

A further advantageous and preferred embodiment is characterized in that each OBOGS unit and/or set of oxygen masks is connected with at least one Chemical Oxygen Generator (COG) and/or Gaseous Oxygen Storage. Both, COGs and gaseous oxygen storage are known. In such an embodiment it becomes possible to temporarily use the additional oxygen sources in case an OBOGS unit suffers a defect until two adjacent OBOGS units are connected or sharing of oxygen masks by the inhabitants is organized.

According to a further aspect of the invention, the object outlined in the introducing portion is solved by a civil aircraft of the aforementioned type in that it comprises at least one oxygen supply device according to one of the above-mentioned preferred embodiments, wherein the cabin is divided in at least two single zones, each zone including a substantially similar number of seats for inhabitants, wherein each OBOGS unit and/or set of oxygen masks is dedicated to one single zone. Each zone forms an autonomous area, supplied with oxygen from the dedicated OBOGS unit connected with the dedicated set of oxygen masks. The dedicated OBOGS unit for one zone is preferably arranged in that zone, i.e. in or above a ceiling portion of the cabin of the aircraft.

According to a first preferred embodiment of the aircraft, the at least two zones and are interconnected in that a first OBOGS unit in a first zone is adapted to supply oxygen to oxygen masks connected to a second OBOGS unit in a second zone in case of failure of the second OBOGS. Preferably each zone is connected to at least two neighbouring zones. Due to this arrangement, in the event of failure of one or more OBOGS units, the associated zones can be supplied with sufficient oxygen from the neighbouring zones. The interconnection between the zones may be realised in that the associated OBOGS units are connected to supply oxygen from one OBOGS unit to another OBOGS unit as outlined above.

In one preferred embodiment, the zones are arranged in a row and each zone extending from sidewall to sidewall of the aircraft cabin. According to this embodiment, a first zone may include rows one to six, a second zone rows seven to 12, a third zone rows 13 to 18 etc. This leads to a simple arrangement and compact autonomous zones. It becomes easy for the cabin crew to figure out where a zone starts and where it ends.

Alternatively and preferred the zones are arranged side to side and extending from the front to the back of the aircraft cabin. According to this embodiment, a first zone would include seats A, second zone seats B, a third zone seats C etc. Such zones would be very narrow and long. However, if the OBOGS unit in one zone suffers from a failure, the inhabitants may easily share their oxygen mask with their seating neighbour.

Preferably the zones are arranged side to side and in a row, so that they form a chessboard like pattern. This arrangement is a mixture of the above two arrangement. Advantageously according to this embodiment every zone comprises at least to adjacent zones to be interlinked with. Preferably the zones are equally distributed over the whole aircraft so that the weight distribution in the aircraft may also be optimized.

Preferably each zone includes about 20 to 30 passenger seats. Even though the size of the zones strongly depends on the power of the involved OBOGS units and of course of the size of the civil aircraft, a number between 20 to 30 seats is advantageous. Particular preferred are about 15 to 18 seats.

Further it is preferred that the aircraft comprises at least five, preferably at least ten zones. As well as the number of seats per zone, also the total number of zones varies with the size of the aircraft. E.g. an aircraft with only ten seats would probably not need five zones. However, five zones is an advantageous number in case one OBOGS unit suffers a defect and cannot produce the desired amount of oxygen. In such a case the lasting four OBOGS unit need to replace the fifth, which is easily possible.

It is further preferred that wherein a first and a third oxygen mask associated to a respective first and third seat of a row of seats is supplied out of the first OBOGS unit and a second and a fourth oxygen mask associated to a respective second and fourth seat of said row of seats is supplied out of the second OBOGS unit, wherein the second seat is positioned between the first and the third seat and the third seat is positioned between the second and the fourth seat. By this, in case of failure of one OBOGS unit, a passenger may use the oxygen mask if the neighbour seat or share this with his seat neighbour, respectively.

In a further aspect of the invention the above object is solved with an OBOGS unit adapted and designed to be used in a device according to claim 1 and a civil aircraft according to claim 8. Such OBOGS units can be advantageously used in oxygen supply devices in civil aircrafts.

For a more complete understanding of the invention, the invention will now be described in detail with reference to the accompanying drawing. The detailed description will illustrate and describe what is considered as a preferred embodiment of the invention. It should of course be understood that various modifications and changes in form or detail could readily be made without departing from the spirit of the invention. It is therefore intended that the invention may not be limited to the exact form and detail shown and described herein, nor to anything less than the whole of the invention disclosed herein and as claimed hereinafter. Further the features described in the description, the drawing and the claims disclosing the invention may be essential for the invention considered alone or in combination. In particular, any reference signs in the claims shall not be construed as limiting the scope of the invention. The wording "comprising" does not exclude other elements or steps. The wording "a" or "an" does exclude a plurality. The wording "a number of" items, comprises also the number one, i. e. a single item, and further numbers like two, three, four and so forth.

The drawings show in:
- Fig. 1: A simplified scheme of a preferred embodiment of an oxygen supply device with two OBOGS units and two sets of oxygen masks to elucidate a working principle;
- Fig. 2: A simplified scheme of a preferred embodiment of an oxygen supply device with three OBOGS units and three sets of oxygen masks;
- Fig. 3: A simplified scheme of a preferred embodiment of an oxygen supply device with two OBOGS units and two sets of oxygen masks, wherein the OBOGS units are interconnected; and
- Fig. 4: A top view of an aircraft cabin of a civil aircraft with multiple zones and oxygen supply devices indicated.

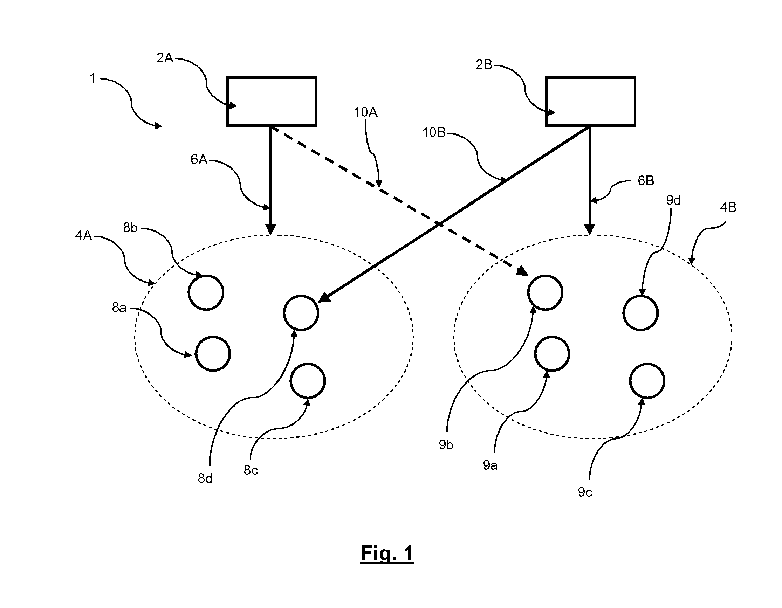

Fig. 1 shows a simplified scheme of an oxygen supply device 1. The device 1 comprises a first OBOGS unit 2A and a second OBOGS unit 2B. The first OBOGS unit 2A is connected to a first set of oxygen masks 4A via a connection line 6A. In the same manner the second OBOGS unit 2B is connected to a second set of oxygen masks 4B via a connection line 6B. In each set of oxygen masks 4A, 4B four masks 8a, 8b, 8c, 8d and 9a, 9b, 9c, 9d are shown. It should be understood that only for the sake of simplicity four masks 8a, 8b, 8c, 8d and 9a, 9b, 9c, 9d are shown in Fig. 1. In practice it would also be possible and preferred to have 20 and more masks in one set of oxygen masks. Usually every seat for an inhabitant inside an aircraft is associated or equipped with one oxygen mask. Further every row has additional masks in case a child is placed at its parent's seat or one mask is defect.

The OBOGS units 2A, 2B and the associated set of masks 4A, 4B are preferably dedicated to a specific zone defined inside an aircraft cabin. For example the first OBOGS unit 2A with the first set of masks 4A is dedicated to the front half of the cabin and the second OBOGS unit 2B with the second set of masks 4B is dedicated to the backmost half of the cabin.

In Fig. 1 the connection lines 6A, 6B are shown to connect the OBOGS unit 2A, 2B with the set 4A, 4B in total. This means, that the connection line between the OBOGS unit 2A, 2B and the masks 8a, 8b, 8c, 8d and 9a, 9b, 9c, 9d can be carried out in a conventional way. The connection line 6A, 6B could be implemented by separate lines running each from the OBOGS unit 2A, 2B to the specific mask 8a, 8b, 8c, 8d and 9a, 9b, 9c, 9d in the associated set 4A, 4B. Alternatively a common line may run from the OBOGS unit 2A, 2B to the set 4A, 4B and then branch into shorter lines to the specific masks 8a, 8b, 8c, 8d and 9a, 9b, 9c, 9d.

According to the invention each mask 8a, 8b, 8c, 8d and 9a, 9b, 9c, 9d is only connected to one OBOGS unit 2A, 2B. In the embodiment according to Fig. 1 the mask 8d out of the first set 4A is connected to the second OBOGS unit 2B via a connection line 10B. The mask 8d is only connected to OBOGS unit 2B even though it is included in the first set 4A. In the same way the mask 9b out of the second set 4B can be connected to the first OBOGS unit 2A via a connection line 10A, which is shown in ghost line in Fig. 1. Further it should be understood that it is also possible that a group of masks out of the first set 4A is connected to the second OBOGS unit 2B and a group of masks out of the second set 4B is connected to the first OBOGS unit 2A. Due to this arrangement the masks 8a, 8b, 8c, 8d and 9a, 9b, 9c, 9d may be provided in a cogged manner between the two sets 4A, 4B.

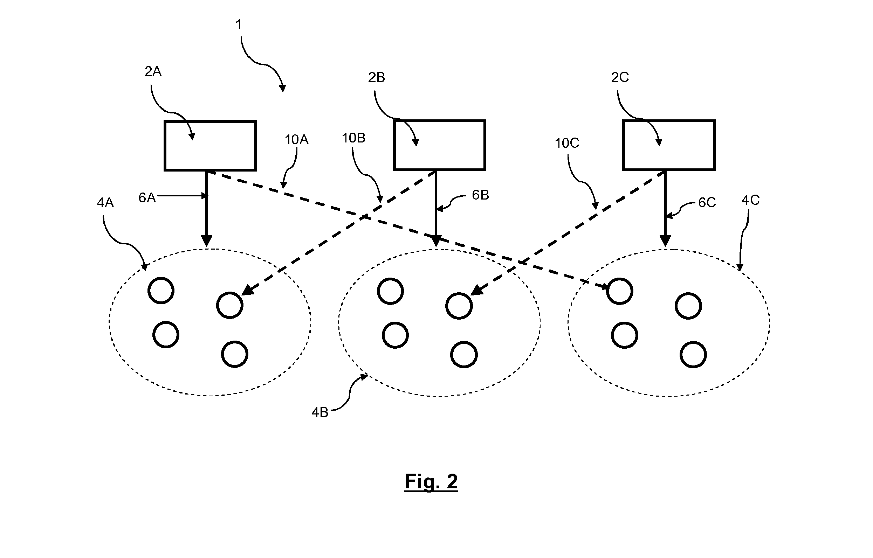

According to Fig. 2 an oxygen supply device 1 is shown, comprising three OBOGS units 2A, 2B, 2C each connected with a set of oxygen masks 4A, 4B, 4C. Each set of oxygen masks 4A, 4B, 4C comprises a plurality of oxygen masks, which are not indicated with reference signs in Fig. 2. However, the embodiment according to Fig. 2 comprises multiple common elements with the embodiment according to Fig. 1. Identical and similar elements are indicated with identical reference signs. Reference is also made to the above description of the embodiment of Fig. 1.

The difference between Fig. 1 and Fig. 2 is that, in Fig. 2 three OBOGS units 2A, 2B, 2C and accordingly three sets of oxygen masks 4A, 4B, 4C are comprised. This should indicate that even three or more OBOGS units and sets may be used in one oxygen supply device 1 according to the present invention.

According to Fig. 2 each OBOGS unit 2A, 2B, 2C is connected to its associated set of masks 4A, 4B, 4C via a connecting line 6A, 6B, 6C. Further, at least one mask out of the first set 4A is connected to the second OBOGS unit 2B, at least one mask out of the second set 4B is connected to the third OBOGS unit 2C and at least one mask out of the third set 4C is connected to the first OBOGS unit 2A. This cogged arrangement of masks and OBOGS units leads to an improved overall oxygen supply to the inhabitants of an aircraft.

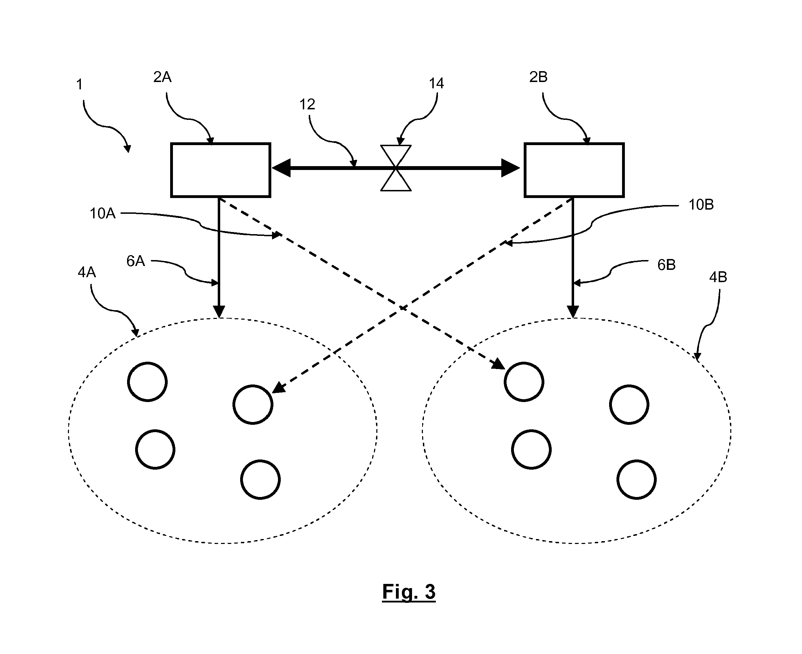

A third embodiment of the oxygen supply device 1 is shown in Fig. 3. The oxygen supply device 1 comprises a first OBOGS unit 2A connected with a first set of oxygen masks 4A via a first connection line 6A ad a second OBOGS unit 2B connected with a second set 4B via a seconds connection line 6B. Again, reference is made to the above description of the embodiments according to Figs. 1 and 2, since the embodiment according Fig. 3 comprises many identical and similar elements. Different to the both above embodiments, the first and second OBOGS units 2A, 2B are connected via a line 12 for supplying oxygen from the first OBOGS unit 2A to the second OBOGS unit 2B and vice versa, as the arrows indicate. This connection 12 is used to supply oxygen from one OBOGS unit 2A, 2B to the other OBOGS unit 2A, 2B in case one of the OBOGS units 2A, 2B suffers from a defect and cannot produce a sufficient amount of oxygen.

The connection line 12 comprises a valve 14. The valve 14 is used to set the both OBOGS units 2A, 2B into communication. The valve 14 may be operated and activated by hand or via an automated activation system comprising a sensor and an activator. The sensor should sense a defect of the OBOGS unit and send a signal to the activator to open the valve 14.

Further, as indicated by the ghost lines in Fig. 3, at least one oxygen mask out of the first set of oxygen masks 4A may additionally be connected via a connection line 10B with the second OBOGS unit 2B and/or at least one oxygen mask out of the second set of oxygen masks 4B may be connected via a connection line 10A with the first OBOGS unit 2A. With this arrangement redundancies can be implemented which lead to an improved safety of the oxygen supply device 1.

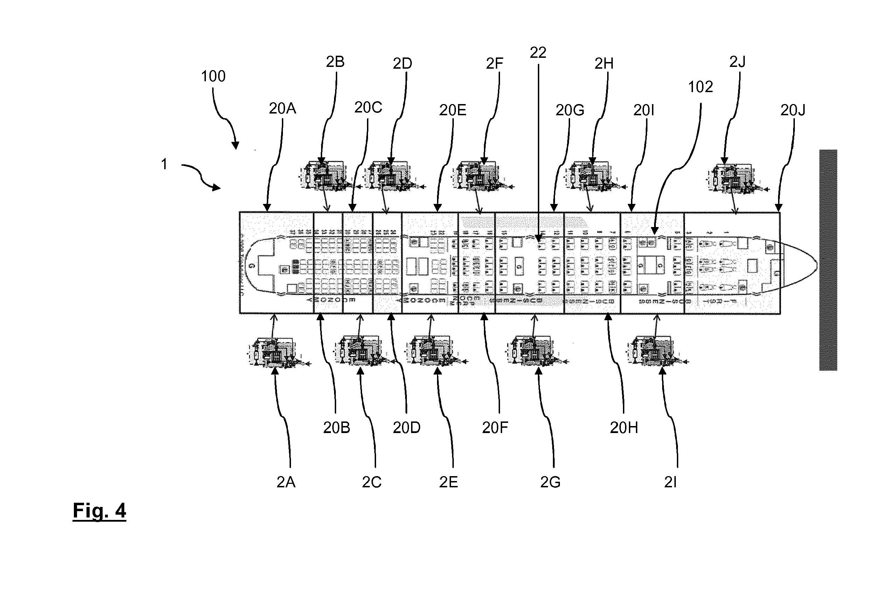

Fig. 4 illustrates the zone arrangement of an aircraft cabin in combination with an oxygen supply device 1 as described above. In Fig. 4 an aircraft 100 comprises an aircraft cabin 102. The cabin 102 is divided into ten zones 20A to 20J. The zones are arranged in a row from the back to the front and extend over the whole width of the aircraft cabin 102. As can be seen in Fig. 4, each zone 20A to 20J comprises a number of seats 22 (for sake of simplicity only one seat is indicated with reference sign). Each zone comprises a substantially similar number of seats 22. For example, zone 20H, a business class zone, comprises 28 seats 22. Zone 20J in the very front of the cabin 102 includes eight seats 22 of the first class, seven seats 22 of the business class and the cockpit.

The aircraft 100 is equipped with an oxygen supply device 1. The oxygen supply device 1 is in general designed according to the above embodiments described with reference to Figs. 2 and 3. The oxygen supply device 1 according to this embodiment (Fig. 4) comprises ten OBOGS units 2A to 2J, so that in each zone 20A to 20J one OBOGS unit 2A to 2J is provided. Each OBOGS unit 2A to 2J is connected with a number of oxygen masks (not shown in Fig. 4) which is at least equal to the number of seats 22 in the specific zone, but normally would be slightly higher due to some additional redundant oxygen masks for safety reasons. The oxygen masks can be provided in a cogged arrangement as described with reference to Fig. 2 above. Additionally the zones 20A to 20J can be interconnected according to the above embodiment described with reference to Fig. 3. In such an interconnection, preferably the adjacent zones are connected. Thus, zone 20A would only be connected to zone 20B, zone 20B itself would be connected to zone 20A and zone 20C etc. Of course different connections can be imagined. It would also be preferred to connect each second zone, thus to connect zone 20A to zone 20C, zone 20B to zone 20D, zone 20C to zone 20A and to zone 20E etc. In total a redundancy is realized leading to an increased safety in oxygen supply for aircraft inhabitants.

## Claims

1. An oxygen supply device (1) for aircraft inhabitants, comprising a first On-Board Oxygen System (OBOGS) unit (2A) connected to a first set of oxygen masks (4A),
**characterized by** a second On-Board Oxygen System (OBOGS) unit (2B) connected to a second set of oxygen masks (4B).

2. The device of claim 1, wherein the second OBOGS unit (2B) is connected to at least one oxygen mask (8d) out of the first set of oxygen masks (4A).

3. The device of claim 2, comprising a third On-Board Oxygen System (OBOGS) unit (2C) connected to a third set of oxygen masks (4C), wherein the third OBOGS unit (2C) is connected to at least one oxygen mask out of the second set of oxygen masks (4B).

4. The device of claim 2 or 3, wherein the second (2B) and/or the third OBOGS unit (2C) is connected to a group of oxygen masks, preferably every second oxygen mask, out of the first (4A) and/or third set of oxygen masks (4C) respectively.

5. The device of claim 1, wherein the first OBOGS unit (2A) is connected to the second OBOGS unit (2B) for supplying oxygen from the first OBOGS unit (2A) to the second OBOGS unit (2B).

6. The device of claim 1, wherein each OBOGS unit (2A to 2J) and/or each set of oxygen masks (4A, 4B, 4C) is dedicated to one defined zone (20A to 20J) inside a cabin (102) of an civil aircraft (100) and wherein each zone (20A to 20J) preferably includes a substantially similar number of seats (22) for inhabitants.

7. The device of claim 1, wherein each OBOGS unit (2A to 2J) and/or set of oxygen masks (4A, 4B, 4C) is connected with at least one Chemical Oxygen Generator (COG) and/or Gaseous Oxygen Storage.

8. A civil aircraft (100), comprising an aircraft inhabitant cabin (102) with a plurality of seats (22) for the aircraft inhabitants, and at least one device (1) of claim 1, **characterized in that** the cabin (102) is divided in at least two single zones (20A to 20J), each zone (20A to 20J) preferably including a substantially similar number of seats (22) for inhabitants, wherein each OBOGS unit (2A to 2J) and/or set of oxygen masks (4A, 4B, 4C) is dedicated to one single zone (20A to 20J).

9. The civil aircraft of claim 8, wherein the at least two zones (20A to 20J) are interconnected in that a first OBOGS unit (2A to 2J) in a first zone (20A to 20J) is adapted to supply oxygen to oxygen masks connected to a second OBOGS unit (2A to 2J) in a second zone (20A to 20J) in case of failure of the second OBOGS unit (2A to 2J).

10. The civil aircraft of claim 9, wherein each zone (20A to 20J) is connected to at least two neighbouring zones (20A to 20J).

11. The civil aircraft of claim 8, wherein the zones (20A to 20J) are arranged in a row and each zone (20A to 20J) extending from sidewall to sidewall of the aircraft cabin (102).

12. The civil aircraft of claim 8, wherein the zones (20A to 20J) are arranged side to side and extending from the front to the back of the aircraft cabin (102).

13. The civil aircraft of claim 8, wherein each zone (20A to 20J) includes about 20 to 30 passenger seats (22).

14. The civil aircraft of claim 8, wherein a first and a third oxygen mask associated to a respective first and third seat of a row of seats is supplied out of the first OBOGS unit and a second and a fourth oxygen mask associated to a respective second and fourth seat of said row of seats is supplied out of the second OBOGS unit, wherein the second seat is positioned between the first and the third seat and the third seat is positioned between the second and the fourth seat.

15. An OBOGS unit (2A to 2J) adapted and designed to be used in a device (1) according to claim 1 and a civil aircraft (100) according to claim 8.
